# EUROPEAN PATENT APPLICATION

(11) **EP 1 988 171 A1**
(43) Date of publication of application: **05.11.2008**
(21) Application number: 06714669.6
(22) Date of filing: 21.02.2006
(51) Int. Cl.: C12P 7/62, A61K 31/225, A61K 45/00, A61P 35/00, C07C 69/604, C12N 1/14

(54) **SUBSTANCE FKI-2342 AND PROCESS FOR PRODUCTION THEREOF**

(71) Applicant: THE KITASATO INSTITUTE, Tokyo 108-8642 (JP)
(72) Inventor: TOMODA, Hiroshi, Chofu-shi Tokyo 182-0034 (JP); OMURA, Satoshi, Tokyo 157-0076 (JP); MASUMA, Rokuro, Tokyo 108-0073 (JP)
(74) Representative: Duckworth, Timothy John
(86) International application number: PCT/JP2006/303530
(87) International publication number: WO 2007/097010

(57) **Abstract**

The present invention relates to FKI-2342 substance which is produced by a process comprising culturing a microorganism belonging to genus Aspergillus and having ability to produce FKI-2342 substance represented by the general formula (I), accumulating FKI-2342 substance in a cultured mass, and isolating FKI-2342 substance from the cultured mass. The obtained FKI-2342 substance is expected to enhance an action of DNA damaging type anticancer agent or radiation therapy and to reduce side effects in the treatment with using the DNA damaging type anticancer agent or the radiation therapy.

## Description

### Technical Field

The present invention relates to a mechanism of repairing DNA damage when DNA of cell is damaged. More particularly, the present invention relates to novel compound FKI-2342, which can increase cancer cell specificity of and sensitivity of a DNA damaging type anticancer agent by inhibiting specifically G₂ checkpoint in the presence of the DNA damaging type anticancer agent and decreasing a side effect, and production thereof.

### Background art

According to the statistical data of the Ministry of Health, Labor and Welfare in 2001, it is reported that cancer is at the top of a cause-specific death rate of the year 2004 in Japan, one out of three persons suffers from cancer in their entire life and one out of four persons dies of cancer. Anticancer agents used for clinical practice in the chemotherapy of cancer are classified into antimetabolites such as 5-FU, alkylating agents such as cisplatin and anticancer agents such as bleomycin and mitomycin, and the like depending on the mode of action and their origin. Among them, alkylating agents and anticancer antibiotics having properties of damaging cellular DNA and cytotoxicity exhibit absolutely clear effect, when they are reached to certain levels, even their action time is short, however they also exhibit attacking action to the normal cells and generate drug adverse action such as anemia, vomiting, depilation, and the like caused by organ and bone marrow damages.

Further, as a result of recent progress of cancer research, various matters have been elucidated. Specifically, difference in DNA repairing mechanisms between the normal cells and the cancer cells has been elucidated. More specifically, when a damage of DNA occurs, DNA can be repaired in G₁ checkpoint and G₂ checkpoint in the normal cells, however DNA is repaired depending on only G₂ checkpoint in many cases of cancer cells. From this fact, if G₂ checkpoint is specifically inhibited in the presence of anticancer agent for damaging DNA, only cancer cells cannot be repaired their DNA, resulting to cell death may occur (Suganuma, et al., Cancer Research, 23: 5887-5891, 1991).

### Disclosure of the invention

Under such circumstance, providing drugs for increasing specificity of anticancer agent to cancer cells is thought to be useful for cancer therapy. More specifically, drugs for increasing specificity of anticancer agent to cancer cells are expected to increase its action and decrease in side effects in the cancer therapy by anticancer agents or radiation with damaging DNA.

As a result of extensive studies on the metabolites produced by microorganisms, we have found that newly isolated microorganism strain FKI-2342 from soil produces substance increasing cancer cell specificity of anticancer agent having property of damaging DNA by an action of inhibiting G₂ checkpoint (hereinafter designates as G₂ checkpoint inhibitor) in the culture liquid. Then active substance was isolated from the culture liquid, and as a result of purification, the substance having chemical structure shown in the formula (I), specifically shown in (II) - (VII) was found. Since such the substances shown in the formula (II) - (VII) are not known in the art, the substances are designated as FKI-2342A substance, FKI-2342B substance, FKI-2342C substance, FKI-2342D substance, FKI-2342E substance and FKI-2342F substance, and these substances are designated as totally FKI-2342 substance.

The present invention is focused to the difference in such the DNA repairing mechanism between the cancer cells and the normal cells, and is intended to provide FKI-2342 substance which has ability to increase specificity of the known anticancer agent against the cancer cells and its production.

The present invention has been completed according to such the knowledge, and provides FKI-2342 substance having the following formula (I): 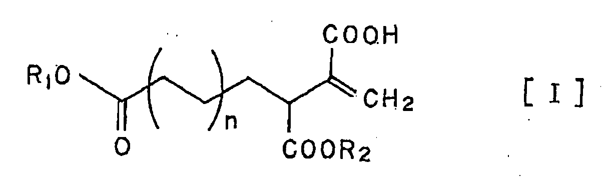 wherein n = 1 - 3, R₁ is CH₃- or CH₃CH₂-, and R₂ is CH₃- or H-.

The present invention provides further FKI-2342A - F substances having following n, R₁ and R₂ in the general formula (I) :

| | n | R₁ | R₂ | Compound No. |
|---|---|---|---|---|
| FKI-2342A | 1 | CH₃- | CH₃- | (II) |
| FKI-2342B | 2 | CH₃- | H- | (III) |
| FKI-2342C | 2 | CH₃CH₂- | H- | (IV) |
| FKI-2342D | 2 | CH₃- | CH₃- | (V) |
| FKI-2342E | 3 | CH₃- | H- | (VI) |
| FKI-2342F | 1 | CH₃CH₂- | H- | (VII) |

Further, the present invention provides FKI-2342 substance shown in the general formula (I) or FKI-2342A - F substances shown in the general formula (II) - (VII), having specific inhibitory action on G₂ checkpoint of cells.

Further, the present invention' provides a method of specifically inhibiting G₂ checkpoint of cancer cells by acting FKI-2342 substance shown in the general formula (I) or FKI-2342A - F substances shown in the general formula (II) - (VII).

Further, the present invention provides a method of producing FKI-2342 substance comprising a microorganism belonging to genus Aspergillus having ability to produce FKI-2342 substance shown in the general formula (I), more specifically FKI-2342A - F substances shown in the general formula (II) - (VII), in a medium, accumulating FKI-2342 substance in the culture mass, and isolating FKI-2342 substance from the culture mass.

Further, the present invention provides a method of producing FKI-2342 substance according to the above wherein the microorganism belonging to genus Aspergillus having ability to produce FKI-2342 substance shown in the general formula (I), more specifically FKI-2342A - F substances shown in the general formula (II) - (VII), is Aspergillus niger FKI-2342 or a mutant thereof.

Further, the present invention provides a strain of Aspergillus niger FKI-2342.

The microorganism having ability to produce FKI-2342 substance shown in the general formula (I), more specifically FKI-2342A - F substances shown in the general formula (II) - (VII) (hereinafter designates as "FKI-2342 producing microorganism") belongs to genus Aspergillus, and can be a strain having ability to produce FKI-2342 substance of the present invention without limitation. Preferable example of the microorganism strain used for production of FKI-2342 substance of the present invention is the strain of Aspergillus niger FKI-2342, which was newly isolated from soil sample of Nagasaki city by the present inventors. Taxonomical properties of the strain are as follows.

### 1. Morphological properties

The strain shows good growth on Czapek yeast extract agar medium, malt extract agar medium and Czapek yeast extract agar medium containing sucrose 20% and bearing of conidia is good.

Microscopic observation of the colony of the strain grown on Czapek yeast extract agar medium shows colorless hyphae with septa. Conidiophore is directly grown on the substrate mycelia with length of 1000 - 2000 × 8.8 - 16 µm, and has reverse T shape foot cells in the basement. Apex of the conidiophore becomes hypertrophied from globose to subglobose with forming vesicle of 32.5 - 50 µm in diameter. Aspergilla are paralleled consisting of metulae (7 - 9 × 3.5 - 5 µm) and ampullar phialides (5.5 - 8 × 3 - 4 µm). Aspergilla cover whole of the vesicle. The pycnidiospore forms from the apex of the phialide to form chain proceed with culturing time. The conidia were globose to aglobose, brown to black, 3 - 4 µm with rough surface.

### 2. Culturing properties

Macroscopic observation of the strain cultured at 25°C for 7 days on various agar media is shown in the following Table.

| Medium | Growth condition on medium (diameter of colony) | Color of surface of colony | Color of reverse of colony | Soluble pigment |
|---|---|---|---|---|
| Czapek yeast extract agar medium | Good (62 - 65 mm) Velvety - powdery Smooth penumbra | Blackish brown | Maize | None |
| Malt extract agar medium | Good (42 - 45 mm) Velvety - powdery Smooth penumbra | Blackish brown | Maize | None |
| Czapek yeast extract agar medium containing sucrose 20% | Good (70 - 75 mm) Velvety - powdery Smooth penumbra | Blackish brown | Maize | None |

In addition, no growth was observed when the present strain was cultured on Czapek yeast extract agar medium at 5°C for 14 days. The strain showed good growth on culturing at 37°C with good bearing of the conidia.

### 3. Physiological properties

### 1) Optimum growth condition

Optimum growth condition of the strain is pH 3 - 8, at 24 - 41°C.

### 2) Growth range

Growth range of the strain is pH 2 - 9, at 14 - 43.5°C.

### 2) Properties on growth

Aerobic growth.

### 4. Conclusion

According to the taxonomical properties, culturing properties and physiological properties of the strain FKI-2342 hereinabove, the present strain was compared with known microorganism strains and was determined to be the strain belonging to genus Aspergillus, specifically Aspergillus niger, and was referred to Aspergillus niger FKI-2342.

### International Deposition

The present strain was deposited as Aspergillus niger FKI-2342 based on Budapest Treaty on the International Recognition of the Deposit of Microorganisms for the Purposes of Patent Procedure in the Incorporated Administrative Agency, National Institute of Technology and Evolution, NITE, Patent Microorganisms Depositary (NPMD) on February 13, 2006 with the accession number NITE ABP-195.

With regard to FKI-2342 substance producing strain used in the present invention, the aforementioned strain Aspergillus niger FKI-2342 can be mentioned as the preferable strain, however taxonomical properties of the microorganism strain are very easy to mutate without maintaining constant properties and are easily mutated by natural or artificial mutation treatments such as conventional ultraviolet irradiation, X-ray irradiation or using mutagenic agents, e.g. N-methyl-N'-nitro-N-nitrosoguanidine, 2-aminopurine, and the like. Artificial mutants obtained by such the treatments as well as strains prepared by cell fusion, strains obtained from gene manipulation and artificial mutation belonging to Aspergillus niger and having ability to produce FKI-2342 substance represented by the formula (II) - (VII) are all able to be used in the present invention.

In practice of the present invention, at first, FKI-2342 producing microorganism belonging to Aspergillus niger is cultured in the medium. Preferable nutrient sources suitable for FKI-2342 substance production which can be used are nutrient medium containing assimilable carbon sources for microorganism, digestible nitrogen sources, further if necessary inorganic salts, vitamins, and the like. Examples of assimilable carbon source are sugars such as glucose, fructose, maltose, lactose, galactose, dextrin, starch, and the like, and vegetable oils such as soybean oil, and the like, and these are used alone or in combination.

Examples of digestible nitrogen sources which can be used alone or in combination are peptone, yeast extract, meat extract, soybean powder, cotton seed powder, corn steep liquor, malt extract, casein, amino acids, urea, ammonium salts, nitrogen salts, and the like. If necessary, salts such as phosphate, magnesium salt, calcium salt, sodium salt, potassium salt, and the like, heavy metal salts such as iron salts, manganese salts, copper salts, cobalt salts, zinc salts, and the like, vitamins and the like, and materials preferable for production of FKI-2342 substance can be added to the medium.

If culturing medium forms heavily foaming, if necessary, liquid paraffin, animal oil, vegetable oil, silicone oil, surface active agents and the like antifoam agents may be added. The above culture medium may be liquid or solid type culture medium if it contains the above nutrient source, and generally the liquid culture medium is used for cultivation. When mass production of the intended substance is performed, as like in the conventional fermentation products, the aeration cultivation is preferred.

When the culture is performed in the large scale tank, in order to prevent delayed growth of the microorganism in the production process, after the production strain is at first inoculated and cultured in the relatively small scale medium, subsequently the cultured mass is transferred into the large scale tank and culturing process is preferably performed. In this case, the composition of the medium used in the preculture and that of the medium used in the main culture may be same or different, and if necessary both can be different in each other.

When the culture is performed in aeration culture, agitation using such as propeller and other machinery means, rotation or shaking in the fermenter, pumping treatment, blowing air, and other known means may be used. Air for aeration for use is sterilized.

Culturing temperature may be changed within ranging the production of FKI-2342 substance by the FKI-2342 substance producing microorganism, and the cultivation is generally performed at 20 - 30°C, preferably around at 27°C. Culturing pH may be generally at pH 5 - 8, preferably at around pH 7. Culturing time depends on the culture condition, and is generally 4 - 7 days. Isolation of FKI-2342 substance from the cultured mass can be performed by such that whole cultured mass is extracted with water miscible organic solvent such as acetone, and the organic solvent is distilled off in vacuo from the extract, then the residue was treated by the extraction with water immiscible organic solvent such as ethyl acetate.

In addition to the above extraction procedure, known method used for isolation of lipid soluble substance such as adsorption chromatography, gel filtration chromatography, thin layer chromatography, centrifugal countercurrent distribution chromatography, high performance liquid chromatography, and the like can be used in combination or repeatedly to isolate and purify FKI-2342 substance.

Physico-chemical properties of FKI-2342 substance are explained hereinbelow.

### FKI-2342A substance

(1) Property: Yellowish syrup
(2) Molecular formula: C₁₁H₁₆O₆

| | |
|---|---|
| HRFAB-MS (m/z) [M+H]⁺: | Calculation 245.1025 |
| | Experimental 245.1024 |

(3) Molecular weight: 244
FAB-MS (m/z) [M⁺]: Experimental 244
(4) UV spectrum: in methanol, λmax(ε) 203 nm (ε 6,000), 205 nm (ε 5,600)
(5) IR spectrum: KBr,
vmax 3450, 2956, 1735, 1438, 1261, 1207 cm⁻¹
(6) Specific rotation: [α]_{D}²⁶ = +1.8 (c = 0.1, MeOH)
(7) Solubility in solvent:
Soluble in methanol, chloroform and ethyl acetate Insoluble in water

(8) Proton and carbon NMR spectrum: In deuterated chloroform, using Varian Inc. 300 MHz NMR spectrometer, chemical shifts of hydrogen and carbon (ppm) are shown as follows. Proviso that: s: singlet, t: triplet, m: multiplet, J: coupling constant

| Position of carbon | chemical shift of carbon (ppm) | chemical shift of hydrogen (ppm) |
|---|---|---|
| C-1 | 170.8 | |
| C-2 | 137.4 | |
| C-3 | 46.1 | 3.50 (1H, t, J = 7.0 Hz) |
| C-4 | 30.4 | 1.72 (1H, m) |
| | | 1.96 (1H, m) |
| C-5 | 22.7 | 1.62 (2H, m) |
| C-6 | 33.6 | 2.34 (2H, t, J = 7.0 Hz) |
| C-7 | 173.7 | |
| C-8 | 129.5 | 5.88 (1H, s) |
| | | 6.51 (1H, s) |
| C-9 | 173.3 | |
| C-10 | 52.2 | 3.69 (3H, s) |
| C-11 | 51.2 | 3.66 (3H, s) |

As a result of detailed examination of various physico-chemical properties and spectral data of FKI-2342A substance, FKI-2342A substance was determined to have the following chemical structure (II):

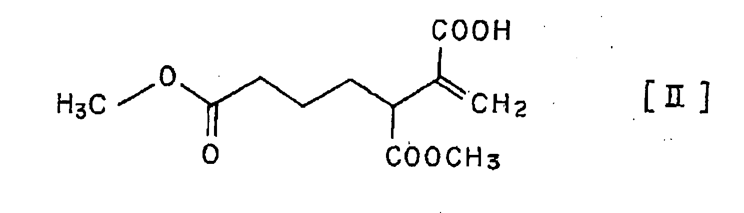

### FKI-2342B substance

(1) Property: Yellowish syrup
(2) Molecular formula: C₁₂H₁₈O₆

| | |
|---|---|
| HRFAB-MS (m/z) [M+H]⁺: | Calculation 259.1182 |
| | Experimental 259.1183 |

(3) Molecular weight: 258
FAB-MS (m/z) [M⁺]: Experimental 258
(4) UV spectrum: in methanol, λmax(ε) 204 nm (ε 8,900), 207 nm (ε 7,900)
(5) IR spectrum: KBr,
vmax 3482, 2952, 1731, 1716, 1438, 1209 cm⁻¹
(6) Specific rotation: [α]_{D}²⁶ = -1.7 (c = 0.1, MeOH)
(7) Solubility in solvent:
Soluble in methanol, chloroform and ethyl acetate Insoluble in water

(8) Proton and carbon NMR spectrum: In deuterated chloroform, using Varian Inc. 300 MHz NMR spectrometer, chemical shifts of hydrogen and carbon (ppm) are shown as follows. Proviso that: s: singlet, t: triplet, m: multiplet, J: coupling constant

| Position of carbon | chemical shift of carbon (ppm) | chemical shift of hydrogen (ppm) |
|---|---|---|
| C-1 | 171.5 | |
| C-2 | 137.5 | |
| C-3 | 46.8 | 3.46 (1H, t, J = 7.0 Hz) |
| C-4 | 30.3 | 1.71 (1H, m) |
| | | 1.92 (1H, m) |
| C-5 | 27.2 | 1.32 (2H, m) |
| C-6 | 29.0 | 1.32 (2H, m) |
| C-7 | 24.9 | 1.61 (2H, m) |
| C-8 | 34.2 | 2.31 (2H, t, J = 7.5 Hz) |
| C-9 | 174.6 | |
| C-10 | 129.9 | 5.86 (1H, s) |
| | | 6.51 (1H, s) |
| C-11 | 179.2 | |
| C-12 | 51.8 | 3.66 (3H, s) |

As a result of detailed examination of various physico-chemical properties and spectral data of FKI-2342B substance, FKI-2342B substance was determined to have the following chemical structure (III):

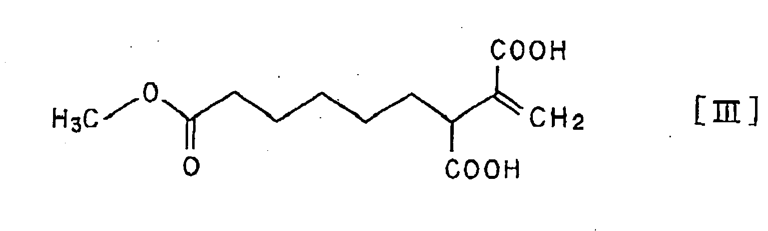

### FKI-2342C substance

(1) Property: Yellowish syrup
(2) Molecular formula: C₁₃H₂₀O₆

| | |
|---|---|
| HRFAB-MS (m/z) [M+H]⁺: | Calculation 273.1338 |
| | Experimental 273.1335 |

(3) Molecular weight: 272
FAB-MS (m/z) [M⁺]: Experimental 272
(4) UV spectrum: in methanol, λmax(ε) 201 nm (ε 7,700), 206 nm (ε 4,500)
(5) IR spectrum: KBr,
vmax 3448, 2931, 1731, 1716, 1261, 802 cm⁻¹
(6) Specific rotation: [α]_{D}²⁶ = -6.0 (c = 0.1, MeOH)
(7) Solubility in solvent:
Soluble in methanol, chloroform and ethyl acetate Insoluble in water

(8) Proton and carbon NMR spectrum: In deuterated chloroform, using Varian Inc. 300 MHz NMR spectrometer, chemical shifts of hydrogen and carbon (ppm) are shown as follows. Proviso that: s: singlet, t: triplet, m: multiplet, J: coupling constant

| Position of carbon | chemical shift of carbon (ppm) | chemical shift of hydrogen (ppm) |
|---|---|---|
| C-1 | 171.2 | |
| C-2 | 137.3 | |
| C-3 | 46.8 | 3.37 (1H, t, J = 7.5 Hz) |
| C-4 | 29.8 | 1.67 (1H, m) |
| | | 1.86 (1H, m) |
| C-5 | 27.0 | 1.28 (2H, m) |
| C-6 | 28.7 | 1.28 (2H, m) |
| C-7 | 24.7 | 1.56 (2H, m) |
| C-8 | 34.2 | 2.24 (2H, t, J = 7.5 Hz) |
| C-9 | 173.9 | |
| C-10 | 129.7 | 5.78 (1H, s) |
| | | 6.45 (1H, s) |
| C-11 | 179.0 | |
| C-12 | 60.3 | 4.05 (2H, q, J = 7.0 Hz) |
| C-13 | 14.2 | 1.18 (3H, t, J = 7.0 Hz) |

As a result of detailed examination of various physico-chemical properties and spectral data of FKI-2342C substance, FKI-2342C substance was determined to have the following chemical structure (IV):

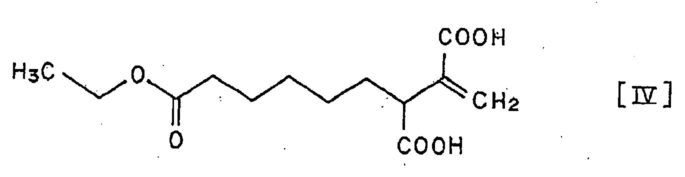

### FKI-2342D substance

(1) Property: Yellowish syrup
(2) Molecular formula: C₁₃H₂₀O₆

| | |
|---|---|
| HRFAB-MS (m/z) [M+H]⁺: | Calculation 273.1338 |
| | Experimental 273.1342 |

(3) Molecular weight: 272
FAB-MS (m/z) [M⁺]: Experimental 272
(4) UV spectrum: in methanol, λmax(ε) 202 nm (ε 17,000), 205 nm (ε 7,700)
(5) IR spectrum: KBr,
vmax 3455, 2952, 2861, 1737, 1438, 1207 cm⁻¹
(6) Specific rotation: [α]_{D}²⁶ = +3.0 (c = 0.1, MeOH)
(7) Solubility in solvent:
Soluble in methanol, chloroform and ethyl acetate Insoluble in water

(8) Proton and carbon NMR spectrum: In deuterated chloroform, using Varian Inc. 300 MHz NMR spectrometer, chemical shifts of hydrogen and carbon (ppm) are shown as follows. Proviso that: s: singlet, t: triplet, m: multiplet, J: coupling constant

| Position of carbon | chemical shift of carbon (ppm) | chemical shift of hydrogen (ppm) |
|---|---|---|
| C-1 | 170.8 | |
| C-2 | 137.7 | |
| C-3 | 46.0 | 3.43 (1H, t, J = 7.0 Hz) |
| C-4 | 30.8 | 1.63 (1H, m) |
| | | 1.84 (1H, m) |
| C-5 | 26.9 | 1.28 (2H, m) |
| C-6 | 28.6 | 1.28 (2H, m) |
| C-7 | 24.5 | 1.56 (2H, m) |
| C-8 | 33.8 | 2.24 (2H, t, J = 7.0 Hz) |
| C-9 | 174.2 | |
| C-10 | 128.8 | 5.79 (1H, s) |
| | | 6.43 (1H, s) |
| C-11 | 173.6 | |
| C-12 | 52.0 | 3.63 (3H, s) |
| C-13 | 51.4 | 3.60 (3H, s) |

As a result of detailed examination of various physico-chemical properties and spectral data of FKI-2342D substance, FKI-2342D substance was determined to have the following chemical structure (V):

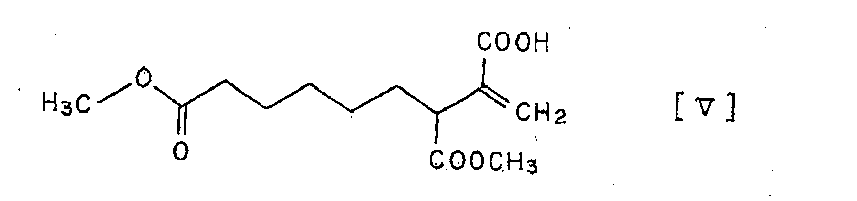

### FKI-2342E substance

(1) Property: Yellowish syrup
(2) Molecular formula: C₁₄H₂₂O₆

| | |
|---|---|
| HRFAB-MS (m/z) [M+H]⁺: | Calculation 287.1495 |
| | Experimental 287.1498 |

(3) Molecular weight: 286
FAB-MS (m/z) [M⁺]: Experimental 286
(4) UV spectrum: in methanol, λmax(ε) 198 nm (ε 13,000)
(5) IR spectrum: KBr,
vmax 3482, 2933, 2857, 1716, 1438, 1203 cm⁻¹
(6) Specific rotation: [α]_{D}²⁶ = +4.9 (c = 0.1, MeOH)
(7) Solubility in solvent:
Soluble in methanol, chloroform and ethyl acetate Insoluble in water

(8) Proton and carbon NMR spectrum: In deuterated chloroform, using Varian Inc. 300 MHz NMR spectrometer, chemical shifts of hydrogen and carbon (ppm) are shown as follows. Proviso that: s: singlet, t: triplet, m: multiplet, J: coupling constant

| Position of carbon | chemical shift of carbon (ppm) | chemical shift of hydrogen (ppm) |
|---|---|---|
| C-1 | 170.9 | |
| C-2 | 137.3 | |
| C-3 | 47.0 | 3.41 (1H, t, J = 7.0 Hz) |
| C-4 | 29.7 | 1.73 (1H, m) |
| | | 1.92 (1H, m) |
| C-5 | 27.2 | 1.31 (2H, m) |
| C-6 | 28.9 | 1.31 (2H, m) |
| C-7 | 29.0 | 1.31 (2H, m) |
| C-8 | 29.0 | 1.31 (2H, m) |
| C-9 | 24.9 | 1.61 (2H, m) |
| C-10 | 34.1 | 2.30 (2H, t, J = 7.5 Hz) |
| C-11 | 174.4 | |
| C-12 | 129.5 | 5.84 (1H, s) |
| C-13 | 178.5 | 6.52 (1H, s) |
| C-14 | 51.5 | 3.67 (3H, s) |

As a result of detailed examination of various physico-chemical properties and spectral data of FKI-2342E substance, FKI-2342E substance was determined to have the following chemical structure (VI):

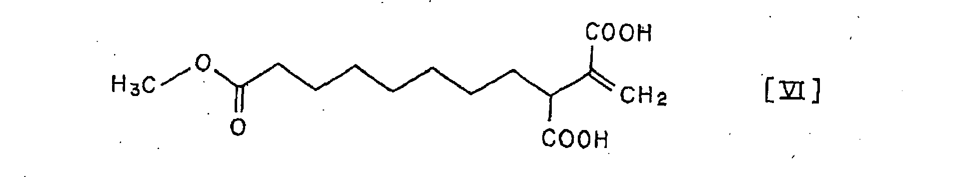

### FKI-2342F substance

(1) Property: Yellowish syrup
(2) Molecular formula: C₁₁H₁₆O₆

| | |
|---|---|
| HRFAB-MS (m/z) [M+H]⁺: | Calculation 245.1025 |
| | Experimental 245.1034 |

(3) Molecular weight: 244
FAB-MS (m/z) [M⁺]: Experimental 244
(4) UV spectrum: in methanol, λmax(ε) 199 nm (ε 11,000)
(5) IR spectrum: KBr,
vmax 3442, 2956, 1716, 1444, 1243, 1203 cm⁻¹
(6) Specific rotation: [α]_{D}²⁶ = 3.1 (c = 0.1, MeOH)
(7) Solubility in solvent:
Soluble in methanol, chloroform and ethyl acetate Insoluble in water

(8) Proton and carbon NMR spectrum: In deuterated chloroform, using Varian Inc. 300 MHz NMR spectrometer, chemical shifts of hydrogen and carbon (ppm) are shown as follows. Proviso that: s: singlet, t: triplet, m: multiplet, J: coupling constant

| Position of carbon | chemical shift of carbon (ppm) | chemical shift of hydrogen (ppm) |
|---|---|---|
| C-1 | 171.3 | |
| C-2 | 136.8 | |
| C-3 | 47.0 | 3.42 (1H, t, J = 7.0 Hz) |
| C-4 | 29.0 | 1.80 (1H, m) |
| | | 1.98 (1H, m) |
| C-5 | 22.7 | 1.68 (2H, m) |
| C-6 | 33.8 | 2.34 (2H, m) |
| C-7 | 173.2 | |
| C-8 | 130.2 | 5.88 (1H, s) |
| | | 6.54 (1H, s) |
| C-9 | 178.7 | |
| C-10 | 60.4 | 4.13 (2H, q, J = 7.0 Hz) |
| C-11 | 14.2 | 1.25 (3H, t, J = 7.0 Hz) |

As a result of detailed examination of various physico-chemical properties and spectral data of FKI-2342F substance, FKI-2342F substance was determined to have the following chemical structure (VII): 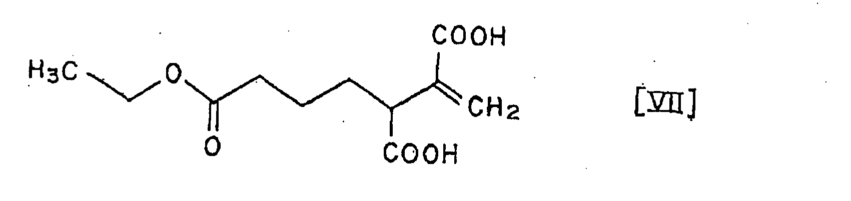 Biological properties of FKI-2342 substance will be explained hereinbelow.

An assay method of G₂ arrest activity with treatment of anticancer agent using cancer cells is performed according to the method of Suganuma et al. (Cancer Research, 23: 5887-5891, 1999).

### 1. Inhibitory action for G₂ checkpoint using Jurkat cells Cell culture and evaluation method

Jurkat cells derived from human lymphoma were cultured in RPMI 1640 medium (a medium adding an inactivated fetal bovine serum (JRH Bioscience Inc.) 50 ml and a mixture 50 ml of penicillin (10,000 units/ml) and streptomycin (10 mg/ml) (Invitrogen Inc.) in RPMI 1640 (IWAKI Co., Ltd.) 500 ml) by using a 75 cm³ culture flask. After centrifugation of the Jurkat cells at 1, 000 rpm for 10 minutes, the supernatant was removed by using aspirator to adjust the number of cells to be 5 × 10⁵ cells/ml with addition of RPMI 1640 medium. Bleomycin (Nippon Kayaku Co., Ltd.) or colchicines (Sigma Inc.) was added to be the final concentration of 40 µM or 0.5 µM, respectively, into the cell suspension. The suspension was added into each well of 96 well multiplate (Corning Corp., the U.S.), which was previously air dried after adding FKI-2342A - F substances 200 µl (1 × 10⁵ cells/well) in each well, and incubated at 37°C for 20 hours.

### Measuring method of cell cycle using FACS

Cells after culturing for 20 hours were centrifuged at 1,000 rpm for 10 minutes, and the supernatant was removed by using aspirator. Krishan's reagent (trisodium citrate (Wako Pure Chemical Industries, Ltd.) 500 ng, IGEPALCA-630 (Sigma Inc.) 1.5 ml, ribonuclease A (Sigma Inc.) 10 mg and propydium iodide (Sigma Inc.) 25 mg dissolved in sterilized water 50.0 ml) 200 µm was added to each well. The well was covered with aluminum foil for light shielding and allowed to stand at room temperature for 1 hour with gently shaking by the shaker to perform DNA staining. Measurement of cell cycle was performed by measuring 10,000 cells using FACS Caliber (Becton Dickinson Inc.) to calculate ratio of cells in sub G₁, G₁, S, and G₂/M phase by using analytical software Ce 11 Quest.

### Activities

Experimental results in relation to FKI-2342A substance - F substance were obtained by using the index of the G₂/M phase cell. A concentration of substance wherein the number of cells at G₂ phase is reduced to 50% when the number of cells arrested at G₂ phase by treatment with bleomycin is set as 100%, is calculated as IC₅₀ (bleo), and similarly a concentration of substance wherein the number of cells at M phase is reduced to 50% is calculated as IC₅₀ (col), and the specificity to the G₂ phase cells as the selectivity is shown in Table 1.

In FKI-2342 substance, IC₅₀ (bleo) was measured to be 1.2 µg/ml - 100 µg/ml, whereas IC₅₀ (col) was measured to be 10 µg/ml - 200 µg/ml, indicating to be clear that FKI-2342A substance inhibits selectively the G₂ arresting action (G₂ checkpoint) generated by an action of the anticancer agent. Specifically, IC₅₀ (bleo) of FKI-2342D substance and FKI-2342E substance was measured as 25 µg/ml and 1.2 µg/ml, respectively, whereas IC₅₀ (col) was measured as 175 µg/ml and 10 µg/ml, respectively, indicating 7-fold and 8-fold differences between both concentrations. Consequently, both substances became apparent to inhibit G₂ checkpoint very selectively.

**Table 1 (in case of Jurkat cells)**

| Compound | IC₅₀ (µ/ml) | | selectivity |
|---|---|---|---|
| | bleomycin | colchicine | |
| A | 100 | 200 | 2 |
| B | 100 | 200 | 2 |
| C | 100 | 200 | 2 |
| D | 25 | 175 | 7 |
| E | 1.2 | 10 | 8 |
| F | 100 | 200 | 2 |

### 2. Inhibitory action for G₂ checkpoint using HCT-116 cells Cell culture and evaluation method

A cell suspension (15 ml) of HCT-116 cells derived from human colon cancer, adjusted to 1.5 × 10⁵ cells/ml with McCoy's medium, was cultured in McCoy's 5A medium (a medium adding an inactivated fetal bovine serum (FBS) 500 ml and a mixture 5 ml of penicillin (10,000 units/ml) and streptomycin (10 mg/ml) in McCoy's 5A (Invitrogen Inc.) 500 ml) in a 75-cm³ culture flask (Corning Corp., the U.S.) under the atmosphere of 0.5% CO₂ at 37°C, and a subinoculation was performed after 3 days.

HCT-116 cells (500 µl) adjusted to give 1.5×10⁵ cells/ml. were added into each well of a 24-well microplate (IWAKI Co., Japan) and incubated at 37°C for 24 hours. Thereafter, the medium was removed and 250 µl of 27 µM bleomycin or that of 0.5 µM colcicine prepared with McCoy's 5A medium were added in each well, and FKI-2342A substance - FKI-2342F substance dissolved in 0.5% DMSO, each 250 µl, were added (final concentration: bleomycin 13 µM, colchicine 0.25 µM, and 0.25% DMSO). The plate was incubated under 0.5% CO₂ at 37°C for 24 hours. After the incubation, the supernatant was recovered in 1.5 ml Eppendorf tube and stored on ice. Cells on the plate were detached by adding trypsin-EDTA solution (150 µl) and culturing at 37°C for 10 minutes. Immediately after the cells were detached, 300 µl of the medium were added to denature the trypsin, and were mixed with the supernatant which was stored on the ice. The mixture was centrifuged at 1,000 rpm for 10 minutes, and the supernatant was removed by using the aspirator, then Krishan's reagent (200 µl) was added thereto, and after allowing to stand for 1 hour, measurement was performed by using FACS.

### Measurement of cell cycle using FACS

Measurement of cell cycle was performed by measuring 10,000 cells using FACS Caliber (Becton Dickinson Inc.) to calculate ratio of cells in sub G₁, G₁, S, and G₂/M phase by using analytical software cell Quest.

### Activities

Experimental results in relation to FKI-2342A substance - F substance were obtained by using the index of the G₂/M phase cell. A concentration of substance, in which the number of cells at G₂ phase is reduced to 50% when the number of cells arrested at G₂ phase by treatment with bleomycin is' set as 100%, is calculated as IC₅₀ (bleo), and similarly a concentration of substance, in which the number of cells at M phase is reduced to 50%, is calculated as IC₅₀ (col), and the specificity to the G₂ phase cells as the selectivity is shown in Table'2.

In FKI-2342 substance, IC₅₀ (bleo) was measured to be 2 µg/ml - 120 µg/ml, whereas IC₅₀ (col) was measured to be 20 µg/ml - 120 µg/ml, indicating they selectively inhibit the G₂ checkpoint. Among them, specifically, IC₅₀ (bleo) of FKI-2342D substance and FKI-2342E substance was measured as 25 µg/ml and 2 µg/ml, respectively, whereas IC₅₀ (col) was measured as 100 µg/ml and 20 µg/ml, respectively, indicating 4-fold and 10-fold differences in a concentration ratio (IC₅₀ (col) / IC₅₀ (bleo)). Consequently, both substances became apparent to inhibit G₂ checkpoint very selectively.

As described hereinabove, FKI-2342A substance - F substance became apparent to inhibit the G₂ checkpoint selectively against both cells of Jurkat cells derived from human lymphoma and HCT-116 cells derived from human colon cancer. In addition, the activity and the selectivity thereof are superior in FKI-2342E substance.

**Table 2 (in case of HCT-116 cells)**

| Compound | IC₅₀ (µg/ml) | | Selectivity IC₅₀(col) /IC₅₀(bleo) |
|---|---|---|---|
| | bleomycin | colchicine | |
| A | 120 | 180 | 2 |
| B | 120 | 180 | 2 |
| C | 120 | 180 | 2 |
| D | 25 | 100 | 7 |
| E | 2 | 20 | 8 |
| F | 100 | 180 | 2 |

### Best Mode for carrying out the invention

The present invention is described in connection with the following example, however the present invention is not limited within such the example.

### Example

### Cultivation

Seed culture was performed by inoculating one loopful FKI-2342 strain, which was stored in a LcA medium (glucose 0.1%, KH₂PO₄ 0.08%, K₂HPO₄ 0.02%, MgSO₄·7H₂O 0.02%, KCl 0.02%, NaNO₃ 0.2%, yeast extract 0.02% and agar 1.5%), in two 500 ml Erlenmeyer flasks containing each 100 ml of GP medium (glucose 2%, yeast extract 0.2%, MgSO₄·7H₂O 0.05%, polypeptone 0.5%, KH₂PO₄ 0.1% and agar 0.1%), which were sterilized by high pressure steam, and shake culturing at 27°C for 3 days. Main culture was performed by using a 30 L jar fermenter (MITSUWA Co., Ltd., Japan) containing 20 L of medium with inoculating the seed culture liquid to give 1% of the final concentration. A culture condition was performed by aeration culture as follows. Culturing temperature: 27°C; culturing time: 6 days; number of rotation: 250 rpm; and aeration 10 L/minute.

### Isolation and purification

The cultured liquid (20 L) cultured for production for 7 days was extracted with the equal quantity of acetone, filtered in vacuo and the acetone was distilled off. The residue was extracted twice with ethyl acetate 20 L, and the ethyl acetate layer was dehydrated by adding anhydrous sodium sulfate, then dried in vacuo to obtain brownish oily substance 20.4 g. The oily substance was dissolved in a small quantity of chloroform and subjected to silica gel column chromatography (80 g, 39 i.d. × 180 mm). Gradual elution was performed by using a mixture of chloroform - methanol (quantity of each solvent 500 ml, chloroform : methanol = 100 : 0, 100 : 1, 75 : 1, 50 : 1, 25 : 1, 1 : 1, 0 : 100). Activity was confirmed in the eluate fraction of chloroform : methanol = 50 : 1 (Fr. 4), and the fraction was concentrated in vacuo to obtain brownish oily substance 579 mg.

Further, the oily substance was dissolved in methanol and purified by using high performance liquid chromatography (HPLC) with the following condition. A column used was PEGASIL ODS (Senshu Kagaku Co., Ltd. , 20 i. d. × 250 mm), and a concentration of acetonitrile as a solvent (containing 0.05% TFA) was increased from 20% to 55% for 50 minutes. Further, detection was performed in the flow rate 8.0 ml/minute. with wavelength at 210 nm. FKI=2342D substance, FKI-2342E substance and FKI-2342F substance were shown in the peaks at 40.8 minutes, 42.0 minutes and 24.0 minutes, respectively, and were collected repeatedly. The aqueous acetonitrile solution was concentrated in vacuo to obtain the aqueous layer, and the aqueous layer was extracted with ethyl acetate, then concentrated in vacuo to obtain FKI-2342D substance 15.0 mg, FKI-2342E substance 3.89 mg and FKI-2342F substance 6.36 mg.

In addition, Fr. 4-1, Fr. 4-2 and Fr. 4-3 were further purified by using liquid chromatography. For purification of the Fr. 4-1, a column used was PEGASIL ODS (Senshu Kagaku Co., Ltd., 20 i.d. × 250 mm), and detection was performed using 30% acetonitrile (containing 0.05% H₃PO₄) in the flow rate 8.0 ml/minute with wavelength at 210 nm. As a result, the peak eluted at 17 minutes was collected repeatedly, and the aqueous acetonitrile solution was concentrated in vacuo to obtain the aqueous layer, and the aqueous layer was extracted with ethyl acetate, then concentrated in vacuo to obtain FKI-2342A substance 7.35 mg.

Subsequently, for purification of the Fr. 4-2, a column used was PEGASIL ODS (Senshu Kagaku Co., Ltd., 4.6 i.d. × 250 mm), and detection was performed using 35% acetonitrile (containing 0.05% H₃PO₄) in the flow rate 1.0 ml/minute with wavelength at 210 nm. As a result, the peak eluted at 7. 2 minutes was collected repeatedly, and the aqueous acetonitrile solution was concentrated in vacuo to obtain the aqueous layer, and the aqueous layer was extracted with ethyl acetate, then concentrated in vacuo to obtain FKI-2342B substance 47.5 mg.

Finally, for purification of the Fr. 4-3, a column used was PEGASIL ODS (Senshu Kagaku Co., Ltd., 20 i.d. × 250 mm), and detection was performed using 30% acetonitrile (containing 0.05% H₃PO₄) in the flow rate 8.0 ml/minute with wavelength at 210 nm. As a result, the peak eluted at 24 minutes was collected repeatedly, and the aqueous acetonitrile solution was concentrated in vacuo to obtain the aqueous layer, and the aqueous layer was extracted with ethyl acetate, then concentrated in vacuo to obtain FKI-2342C substance 7.36 mg.

### Industrial applicability

The present invention relates to FKI-2342 substance which is produced by a process comprising culturing a microorganism belonging to genus Aspergillus and having ability to produce FKI-2342 substance represented by the general formula (I), accumulating FKI-2342 substance in the cultured mass, and isolating FKI-2342 substance from the cultured mass. The obtained FKI-2342 substance is expected to enhance an action of DNA damaging type anticancer agent or radiation therapy and to reduce side effects in the treatment with using the DNA damaging type anticancer agent or the radiation therapy.

## Claims

1. FKI-2342 substance represented by the general formula (I) 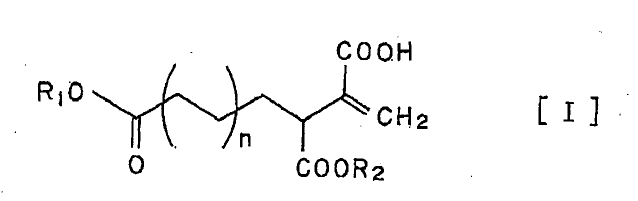 wherein R₁ is CH₃- or CH₃CH₂-, R₂ is CH₃- or H-, and n is 1 to 3.

2. FKI-2342A substance represented by the formula

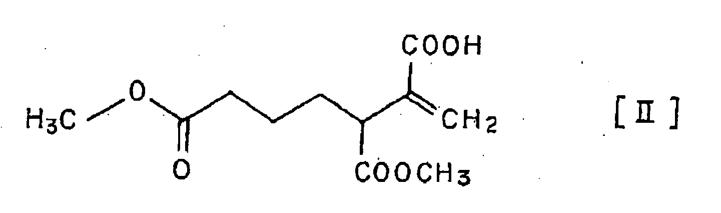

3. FKI-2342B substance represented by the formula

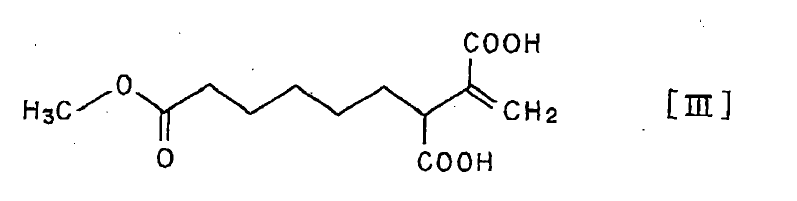

4. FKI-2342C substance represented by the formula

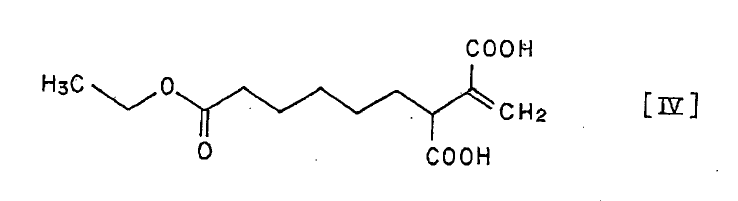

5. FKI-2342D substance represented by the formula

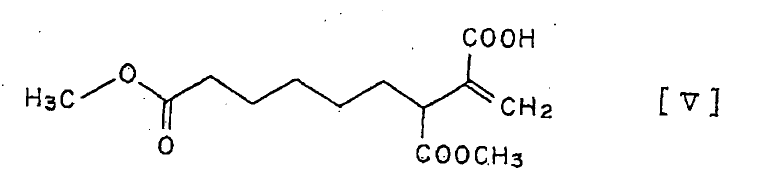

6. FKI-2342E substance represented by the formula

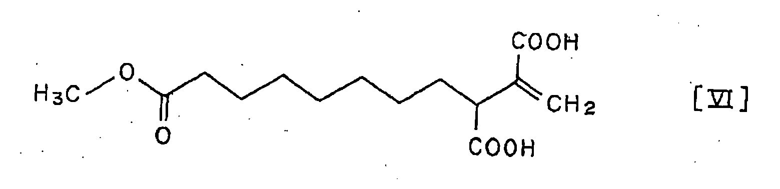

7. FKI-2342F substance represented by the formula

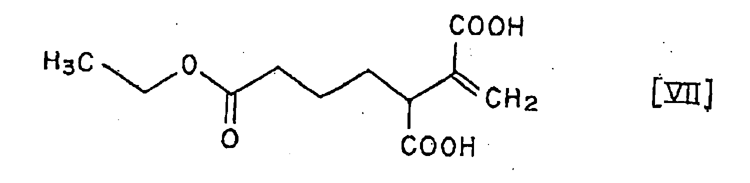

8. A method of specifically inhibiting G₂ checkpoint of cancer cells comprising exerting an action of FKI-2342A substance.

9. The method according to claim 8 comprising exerting an action of FKI-2342A substance in the presence of anticancer agent which damages DNA.

10. A method of specifically inhibiting G₂ checkpoint of cancer cells comprising exerting an action of FKI-2342B substance.

11. The method according to claim 10 comprising exerting an action of FKI-2342A substance in the presence of anticancer agent which damages DNA.

12. A method of specifically inhibiting G₂ checkpoint of cancer cells comprising exerting an action of FKI-2342C substance.

13. The method according to claim 12 comprising exerting an action of FKI-2342C substance in the presence of anticancer agent which damages DNA.

14. A method of specifically inhibiting G₂ checkpoint of cancer cells comprising exerting an action of FKI-2342D substance.

15. The method according to claim 14 comprising exerting an action of FKI-2342D substance in the presence of anticancer agent which damages DNA.

16. A method of specifically inhibiting G₂ checkpoint of cancer cells comprising exerting an action of FKI-2342E substance.

17. The method according to claim 16 comprising exerting an action of FKI-2342E substance in the presence of anticancer agent which damages DNA.

18. A method of specifically inhibiting G₂ checkpoint of cancer cells comprising exerting an action of FKI-2342F substance.

19. The method according to claim 18 comprising exerting an action of FKI-2342F substance in the presence of'anticancer agent which damages DNA.

20. A process for production of FKI-2342 substance comprising culturing a microorganism belonging to genus Aspergillus and having ability to produce FKI-2342 substance represented by the general formula (I) in a medium, accumulating FKI-2342 substance in a cultured mass, and isolating FKI-2342 substance from the cultured mass.

21. A process for production of FKI-2342A, B, C, D, E and/or F substances comprising culturing a microorganism belonging to genus Aspergillus and having ability to produce FKI-2342A, B, C, D, E and/or F substances represented by the general formulae (II) to (VII) in a medium, accumulating FKI-2342A, B, C, D, E and/or F substances in a cultured mass, and isolating FKI-2342A, B, C, D, E and/or F substances from the cultured mass.

22. Aspergillus niger NITE ABP-195.
